# EUROPEAN PATENT APPLICATION

(11) **EP 3 320 884 A1**
(43) Date of publication of application: **16.05.2018**
(21) Application number: 16198773.0
(22) Date of filing: 15.11.2016
(51) Int. Cl.: A61F 9/00, B65D 83/30

(54) **EYE NEBULIZER**

(71) Applicant: Huang, Yu-Kang, Taichung City (TW)
(72) Inventor: Huang, Yu-Kang, Taichung City (TW)
(74) Representative: Horak, Michael

(57) **Abstract**

The eye nebulizer (3) deploys at least a block piece (3333) along a tube (33) for the nebulized medication so as to achieve an S-shaped flow path, thereby preventing the nebulized medication from directly impacting and injuring the eye. In addition, due to the altered and curved flow path, the nebulized medication would diffuse around the rim of the eye cup (32), thereby covering a wider treatment area around the eye. The eye nebulizer (3) therefore achieves an enhanced treatment effect and a greater applicability.

## Description

### BACKGROUND OF THE INVENTION

### (a) Technical Field of the Invention

The present invention is generally related to nebulizers, and more particular to a nebulizer for eye treatment.

### (b) Description of the Prior Art

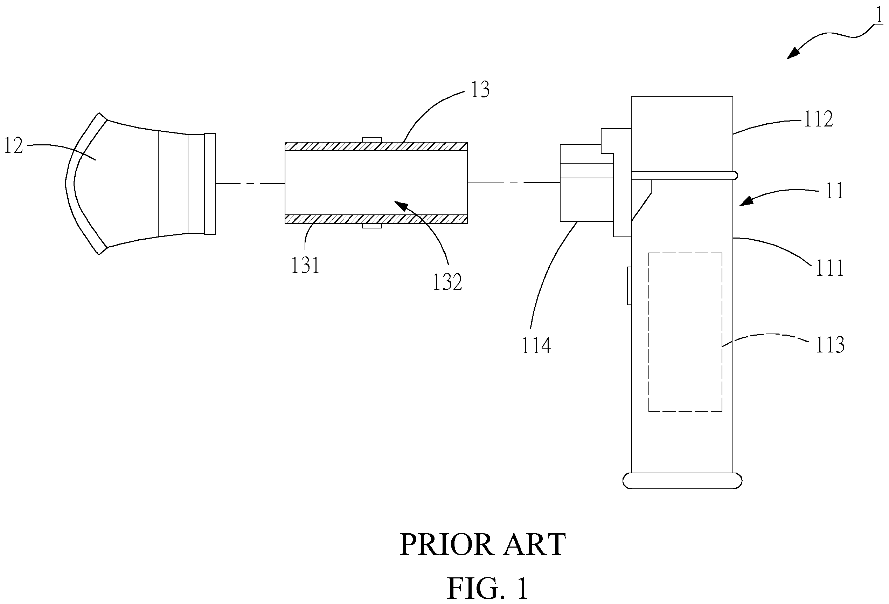

As shown in FIG 1, a conventional eye nebulizer 1 includes a nebulization unit 11, an eye cup 12, and a tube 13 respectively connecting the eye cup 12 and the nebulization unit 11. The nebulization unit 11 includes a main member 111, a reservoir member 112 configured on the main member 111 and storing a medication (not shown), a vibrator module 113 (indicated by the dotted box) inside the main member 111 for nebulizing the medication stored in the reservoir member 112, and a nozzle 114 configured on the main member 111 and connecting the tube 13 to release the nebulized medication. The tube 13 has a channel 132 surrounded by a wall 131 and connecting the nozzle 114 so as to allow the nebulized medication to pass through. To operate the eye nebulizer 1, the eye cup 12 is positioned to cover the eye. The nebulization unit 113 vibrates the medication in the reservoir member 112. The medication is as such gradually nebulized and released from the nozzle 114. The nebulized medication then passes through the channel 132 and applies on the eye within the eye cup 12.

However, the nebulized medication reaches the eye directly and uninterrupted through the nozzle 114 and the tube 13. If the nebulization unit 11 produces too strong a pressure and the nebulized medication would be sprayed towards the eye in a jet stream, causing uncomfortableness or even harm to the eye. Additionally, if a user directly apply the eye nebulizer 1 without checking and removing foreign object or debris attached to the nozzle 114 or the tube 13, the foreign object or debris may impact the eye along with the nebulized medication, thereby injuring the eye.

### SUMMARY OF THE INVENTION

Therefore, an objective of the present invention is to provide a novel eye nebulizer that, by deploying at least a block piece along a tube for the nebulized medication, alters a flow path of the nebulized medication into an S-shaped one, thereby preventing the nebulized medication from directly impacting and injuring the eye. In addition, due to the altered and curved flow path, the nebulized medication would diffuse around the rim of the eye cup, thereby covering a wider treatment area around the eye. The eye nebulizer therefore achieves an enhanced treatment effect and a greater applicability.

The eye nebulizer includes a nebulization unit, an eye cup, and a tube respectively connecting the eye cup and the nebulization unit. The tube includes a channel connecting the nebulization unit and the eye cup, a wall surrounding the channel, and at least a block piece inside the channel configured on the wall and forming an S-shaped flow path for the nebulized medication flowing through the channel. Due to the S-shaped flow path, the nebulized medication would diffuse around the rim of the eye cup, thereby covering a wider treatment area around the eye. The eye nebulizer therefore achieves an enhanced treatment effect and a greater applicability.

The foregoing objectives and summary provide only a brief introduction to the present invention. To fully appreciate these and other objects of the present invention as well as the invention itself, all of which will become apparent to those skilled in the art, the following detailed description of the invention and the claims should be read in conjunction with the accompanying drawings. Throughout the specification and drawings identical reference numerals refer to identical or similar parts.

Many other advantages and features of the present invention will become manifest to those versed in the art upon making reference to the detailed description and the accompanying sheets of drawings in which a preferred structural embodiment incorporating the principles of the present invention is shown by way of illustrative example.

### BRIEF DESCRIPTION OF THE DRAWINGS

FIG 1 is a schematic diagram showing a conventional eye nebulizer.
FIG 2 is a perspective schematic diagram showing an eye nebulizer according to an embodiment using a single block piece with details of a tube of the eye nebulizer revealed.
FIG 3 is a perspective schematic diagram showing an eye nebulizer according to another embodiment using two block pieces and flow paths of nebulized medication of the eye nebulizer.
FIG 4 is a perspective schematic diagram showing an eye nebulizer according to yet another embodiment using two block pieces and flow paths of nebulized medication of the eye nebulizer.
FIGS. 5-1 and 5-2 are perspective diagrams showing still another two embodiments using two fixed block pieces.
FIG 6 is a schematic diagram showing an application scenario of the eye nebulizer according to the present invention.

### DETAILED DESCRIPTION OF THE PREFERRED EMBODIMENTS

The following descriptions are exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

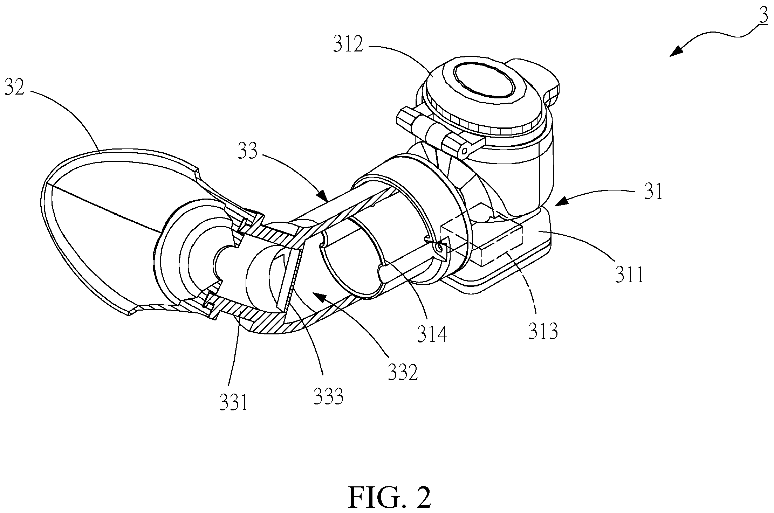

As shown in FIG 2, an eye nebulizer 3 according to an embodiment of the present invention includes a nebulization unit 31, an eye cup 32, and a tube 33 respectively connecting the eye cup 32 and the nebulization unit 31. The nebulization unit 31 includes a main member 311, a reservoir member 312 configured on the main member 311 and storing a medication (not shown), a vibrator module 313 (indicated by the dotted box) inside the main member 311 for nebulizing the medication stored in the reservoir member 312, and a nozzle 314 configured on the main member 311 and connecting the tube 33 to release the nebulized medication.

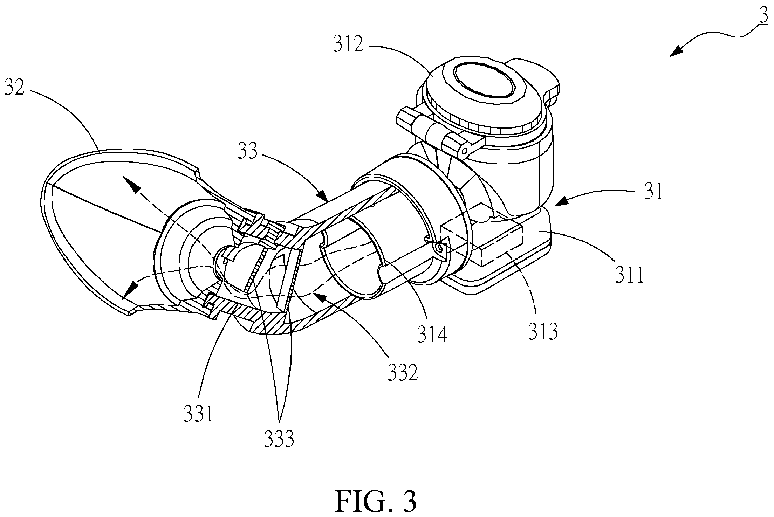

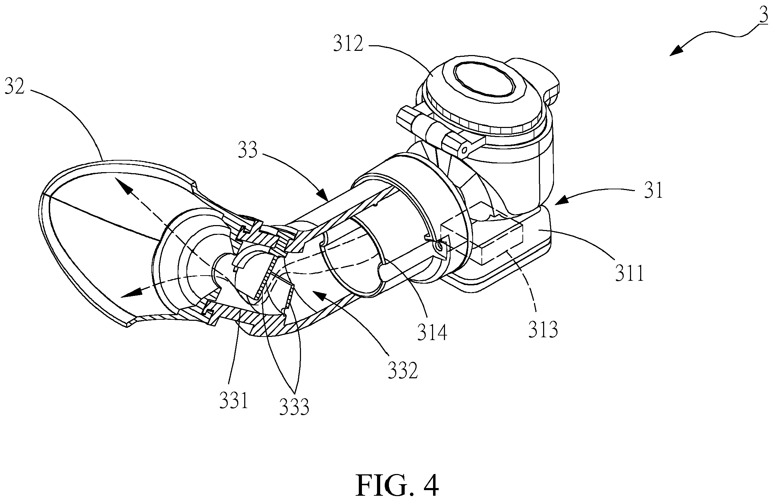

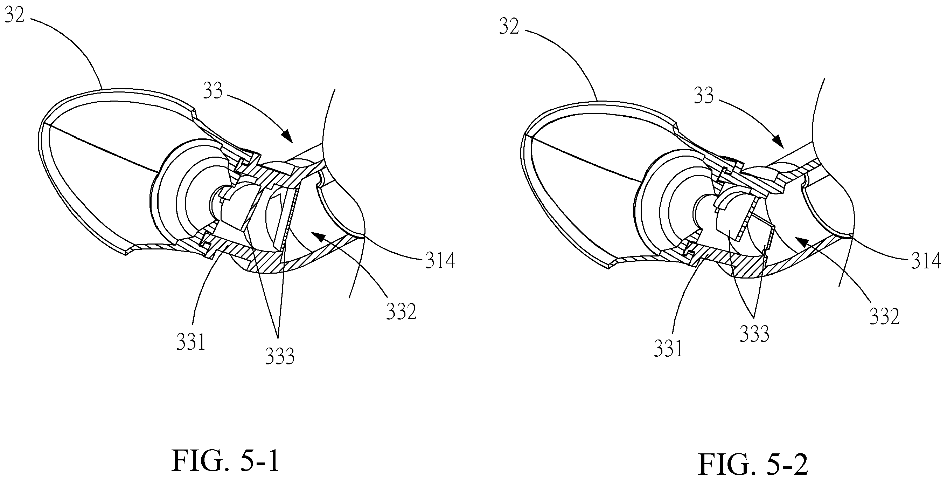

The tube 33 has a channel 332 surrounded by a wall 331 and connecting the nozzle 314 so as to allow the nebulized medication to pass through. The tube 33 has a curved and elongated shape. Inside the channel 332, there is at least a block piece 333 on the wall 331 to interfere and shape a flow path of the nebulized medication passing through the channel 332. As shown in FIGS. 3 and 4, there are two block pieces 333, one fixedly attached to and one detachably plugged into the wall 331. Alternatively, as shown in FIGS. 5-1 and 5-2, the block pieces 333 are all integrally formed with the tube 33 and fixedly configured on the wall 331 By configuring multiple block pieces 333 of various shapes and directions at intervals on the wall 331 along the channel 332, various types of flow paths, such as S-shaped flow paths, may be formed so as to adapt to the different pressures from the nebulization unit 31 and the different amounts of nebulized medications.

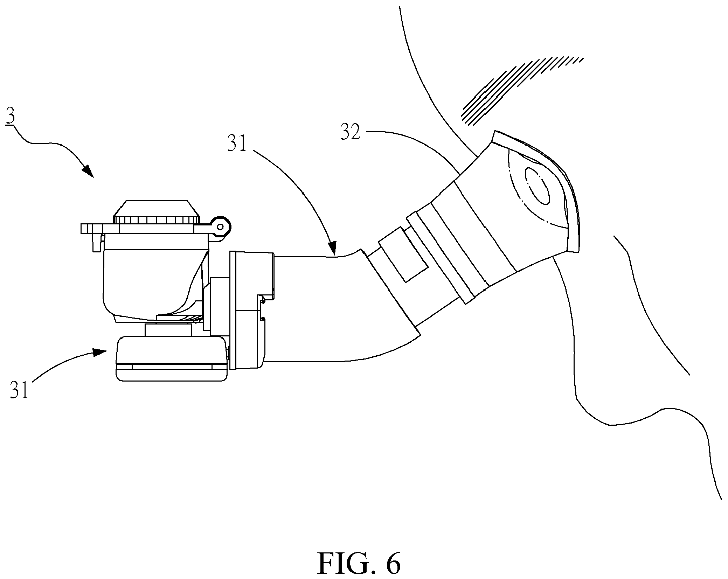

As shown in FIGS. 3 and 6, to operate the eye nebulizer 3, a required medication (or water or saline solution) is first placed in the reservoir member 312. The reservoir member 312 is then joined to the main member 311. The vibrator module 313 is engaged to shake the reservoir member 312. The medication (or water or saline solution) is nebulized into a mist and the mist is sprayed through the nozzle 314. As the mist flows through the tube 33, it is interfered and blocked by the block pieces 333 and then finally applied to the eye covered by the eye cup 32. Due to the curvature of the tube 33 and the alternately configured block pieces 33, a direct flow path of the mist is altered into a curved flow path, thereby preventing the mist from directly impacting and injuring the eye. In addition, due to the altered and curved flow path, the mist would diffuse around the rim of the eye cup 32, thereby covering a wider treatment area around the eye. Foreign object or debris attached to the tube 33 or the nozzle 314 would be blocked by the block pieces 333 and the curved tube 33, again preventing the eye from being injured.

In summary, the eye nebulizer achieves a curved flow path (e.g., an S-shaped flow path) by employing a curved tube and configuring one or more block pieces along the tube so as to protect the eye and cover a wider treatment area.

While certain novel features of this invention have been shown and described and are pointed out in the annexed claim, it is not intended to be limited to the details above, since it will be understood that various omissions, modifications, substitutions and changes in the forms and details of the device illustrated and in its operation can be made by those skilled in the art without departing in any way from the claims of the present invention.

## Claims

1. An eye nebulizer (3), comprising a nebulization unit (31), an eye cup (32), and a tube (33) respectively connecting the eye cup (32) and the nebulization unit (31),
wherein the nebulization unit (31) comprises a main member (311), a reservoir member (312) configured on the main member (311) and storing a medication, a vibrator module (313) inside the main member (311) for nebulizing the medication stored in the reservoir member (312), and a nozzle (314) configured on the main member (311) and connecting the tube (33) to release the nebulized medication; and
the tube (33) comprises a channel (332) connecting the nozzle (314) and the eye cup (32), a wall (331) surrounding the channel (332), and at least a block piece (333) inside the channel (332) configured on the wall (331) and forming an S-shaped flow path for the nebulized medication flowing through the channel (332).

2. The eye nebulizer according to claim 1, wherein the tube (33) has a curved and elongated shape.

3. The eye nebulizer according to claim 1 or claim 2, wherein there are at least two block pieces (333) configured at intervals along the channel (332).

4. The eye nebulizer according to claim 1, wherein at least a block piece (333) is integrally formed with the tube (33) and fixedly attached to the wall (331).

5. The eye nebulizer according to claim 3, wherein at least one of the block pieces (333) is integrally formed with the tube (33) and fixedly attached to the wall (331).

6. The eye nebulizer according to claim 3, wherein at least one of the block pieces (333) is detachably plugged into the wall (331).
